# EUROPÄISCHE PATENTSCHRIFT

(11) **EP 2 320 988 B1**
(45) Veröffentlichungstag und Bekanntmachung des Hinweises auf die Patenterteilung: **05.06.2013**
(21) Anmeldenummer: 09736110.9
(22) Anmeldetag: 28.08.2009
(51) Int. Cl.: A61M 5/14, A61M 31/00, A61J 7/00, A61B 5/145, A61B 5/07

(54) **ELEKTRONISCHE PILLE ZUR STEUERBAREN ABGABE EINER SUBSTANZ, INSBESONDERE EINES MEDIKAMENTS, IN EINEM MENSCHLICHEN ODER TIERISCHEN KÖRPER**
ELECTRONIC PILL FOR DISPENSING A SUBSTANCE, IN PARTICULAR A DRUG, IN A HUMAN OR ANIMAL BODY IN A CONTROLLABLE MANNER
PILULE ÉLECTRONIQUE POUR LA DÉLIVRANCE PILOTABLE D'UNE SUBSTANCE, EN PARTICULIER D'UN MÉDICAMENT, DANS UN CORPS HUMAIN OU ANIMAL

(30) Priorität: 29.08.2008 DE 102008044994
(43) Veröffentlichungstag der Anmeldung: 18.05.2011
(73) Patentinhaber: Hochschule Offenburg, 77652 Offenburg (DE)
(72) Erfinder: JANSEN, Dirk, 77797 Ohlsbach (DE); FAWAZ, Nidal, 72654 Neckardenzlingen (DE)
(74) Vertreter: Eder, Thomas
(86) Internationale Anmeldenummer: PCT/DE2009/001213
(87) Internationale Veröffentlichungsnummer: WO 2010/022716

(56) Entgegenhaltungen:
- WO-A-2006/077528
- WO-A-2008/012700
- US-A- 3 788 322
- US-A- 3 797 492

## Beschreibung

Die Erfindung betrifft eine elektronische Pille zur steuerbaren Abgabe einer Substanz, insbesondere eines Medikaments, in einem menschlichen oder tierischen Körper mit den Merkmalen des Oberbegriffs des Patentanspruchs 1.

Eine elektronische Pille ist ein medizinisches Gerät zur Medikamentenabgabe an Orten im Körper, die durch chemische oder physikalische Kapselung von Medikamenten sonst nicht oder nur unter großen Einschränkungen erreicht werden können. Vorzugsweise werden derartige elektronische Pillen geschluckt oder auf andere Weise in den Körper eingebracht. Weiterhin erschließt die kontrollierte Wirkstoff-Freisetzung neue Therapien, da die Menge des freizusetzenden Wirkstoffs auch nach Einnahme noch kontrolliert und gegebenenfalls interaktiv dosiert werden kann. Durch Verteilung der Freisetzung über einen vorbestimmten Zeitraum in geringen Dosen kann weiterhin die Verträglichkeit des Medikaments erhöht und die Langzeitwirkung unterstützt werden. Durch in der Pille angeordnete Sensoren, z.B. einen PH-Sensor/ Temperatursensor, kann eine gezielte Freisetzung in einem angestrebten Umfeld erfolgen, z. B. im Dünn- oder Dickdarm oder an aufgefundenen entzündlichen Stellen, was entweder automatisch vorprogrammiert oder nach Meldung der Detektion durch das Kontrollgerät initiiert werden kann. Damit wird die Wirksamkeit des Medikaments erhöht.

Die elektronische Pille eignet sich insbesondere zur oralen Dauertherapie bei Diabetes Mellitus, da sie im Dickdarm bis zu 4 Tagen verharrt und dabei als abrufbare Insulinquelle für die Diabetes Therapie dienen kann. Weitere Anwendungen sind beispielsweise die Gabe von Peptiden und sonstigen empfindlichen Medikamenten, die im oberen Teil des Darms zerstört oder nicht resorbiert werden. Elektronische Pillen können auch zur gezielten, zeitlich gestreckten Freisetzung von Medikamenten eingesetzt werden, die bei einer direkten Gabe unverträglich wären.

Es sind elektronische Pillen bekannt, z.B. aus der US 5,279,607, bei denen die Medikamentenabgabe durch ein Funksignal ausgelöst wird, wobei dieses Signal unmittelbar die Freisetzung auslöst. Hierbei wird das gesamte, gespeicherte Medikament auf einmal abgegeben, wenn ein elektrischer Draht durchgebrannt wird.

Weiterhin ist aus der US 2007/0213659 A11 eine elektronische Pille bekannt, die über eine Ultraschallübertragung, einen Timing-Mechanismus und ein steuerbares Ventil verfügen, welches geöffnet und geschlossen werden kann und ein vorprogrammiertes Dosierprofil ablaufen lassen kann sowie über einen separaten RFID Chip verfügt, der zur Identifizierung dient.

Des Weiteren beschreibt die US 2007/0270630 A1 eine elektronische Pille, in der die Medikamentenfreisetzung durch Funk gesteuert durch einen Federmechanismus mit Drahtauslösung erfolgt. Zum Ausstoßen des Medikaments wird ein Kolben verwendet. Eine ähnliche elektronische Pille ist auch in der WO 2006/077528 A2 beschrieben, wobei hier eine Steuereinheit vorgesehen ist, welche wenigstens ein Verschlusselement einer Abgabeöffnung, welche in einen geöffneten und geschlossenen Zustand steuerbar ist, und/oder einen Druckerzeugungsmechanismus, wie eine KolbenZylindereinheit, in einer vorgegebenen Weise ansteuert. Die US 2006/0093663 A1 beschreibt eine Kapsel mit einem piezoelektrischen Aktuator zur Öffnung bzw. Steuerung der Medikamentenfreigabe.

Auch die WO 2006/056944 A1 beschreibt eine elektronisch kontrollierbare Pille und schlägt unter anderem vor, zur Abgabe des Medikaments eine integrierte Pumpe zu verwenden. Die WO 2008/038199 A1 offenbart ebenfalls eine Anordnung mit steuerbarer Pumpe. Das Gehäuse ist bei dieser elektronischen Pille zweiteilig ausgebildet, wobei eine Substanzträgereinheit getrennt von den übrigen Einheiten der Pille vorgesehen ist.

Schließlich beschreibt die US 4,507,115 eine medizinische Kapsel, bei der zum Ausstoß der Substanz ein Kolben verwendet wird, der mittels eines Betätigungselements verschiebbar ist, das aus einem Memory-Metall besteht.

All diese Mechanismen zum Ausstoß der abzugebenden Substanz weisen den Nachteil auf, dass sie entweder sehr aufwändig und damit teuer in der Herstellung sind, oder die Abgabe der Substanz nicht entsprechend dem gewünschten, meist konstanten Massenstrom mit einem vorgegebenen zeitlichen Verlauf ermöglichen.

Bei vielen Anwendungen ist es auch nachteilig, wenn ein einmal begonnener Ausschüttungsvorgang auch nicht mehr zu stoppen ist, wie das bei elektronischen Pillen der Fall ist, bei denen das Ventil nur gesteuert geöffnet, nicht aber wieder geschlossen werden kann.

Ausführungen mit Pumpen ermöglichen zwar die Bereitstellung eines gewünschten zeitlichen Verlaufs der Abgabe der betreffenden Substanz, sind jedoch sehr aufwändig, insbesondere wenn sie piezoelektrisch angetrieben werden. Auch ist dabei hohe Betriebsspannung der Piezoelemente problematisch, da solche Spannungen im Körper ein hohes Risiko beinhalten. Elektromagnetisch angetriebene Ventile sind aus diesem Grund günstiger, aber komplex und teuer in der Fertigung.

Zudem weisen die bekannten Konstruktionen von elektronischen Pillen, die in der Praxis realisiert wurden, nicht die gewünschte Betriebssicherheit und Zuverlässigkeit auf.

Ausgehend von diesem Stand der Technik liegt der Erfindung die Aufgabe zugrunde, eine verschluckbare elektronische Pille zur steuerbaren Abgabe einer Substanz, inbesondere eines Medikaments, in einem menschlichen oder tierischen Körper zu schaffen, einfach aufgebaut und damit einfach und kostengünstig herstellbar ist und die ansteuerbar die Abgabe der Substanz mit einem vorbestimmten Massenstrom ermöglicht.

Die Erfindung löst diese Aufgabe mit den Merkmalen des Patentanspruchs 1.

Die Erfindung geht von der Erkenntnis aus, dass durch die Kombination einer Drosselstrecke, die als Drosselelement ausgebildet ist oder ein Drosselelement umfassen kann, das sich im Abgabepfad für die abzugebende Substanz befindet, und einem einfachen Ventil, welches zwischen einer Geöffnet-Stellung (mit festem Öffnungsquerschnitt) und einer Geschlossen-Stellung hin und her schaltbar ist, so dass auf einfache Weise ein gewünschter Massenstrom der Substanz einstellbar ist. Dieser kann durch die Änderung der Dimensionierung der Drosselstrecke bzw. des Drosselelements an die Substanzeigenschaften, insbesondere deren Viskosität oder Fließfähigkeit angepasst werden. Es können beispielsweise mehrere, sich lediglich hinsichtlich der Drosselstrecke bzw. des Drosselelements unterscheidende Standard-Varianten der elektronischen Pille hergestellt werden, die jeweils verschiedene Anwendungsfälle abdecken, beispielsweise jeweils einen Bereich für die Viskosität der zu verwendenden Substanz. Selbstverständlich kann jedoch auch eine individuelle Herstellung der Pille mit einer individuell angepassten Drosselstrecke bzw. einem individuell angepassten Drosselelement erfolgen.

Die Drosselstrecke bzw. das Drosselelement kann als Keramikelement ausgebildet sein, welches eine definierte Porosität aufweist und damit einen definierten fluidischen Widerstand für die abzugebende Substanz aufweist. Derartige keramische Materialien sind kostengünstig und in vielen Varianten erhältlich. Die Viskosität des Materials wird auf die Substanz abgestimmt und kann typischerweise aus einer Glas-Sinter-Fritte bestehen, die einen definierten Durchfluss im Bereich von beispielsweise 1-20 Mikrolitern/sec bei gegebener Temperatur (z.B. der menschlichen Körpertemperatur von 37°C) und gegebenem Vordruck in der Größenordnung von beispielsweise 0.5-3 bar generiert.

Damit lässt sich der Herstellungsaufwand einer elektronischen Pille für einen gewünschten Anwendungszweck drastisch reduzieren. Es muss lediglich die Drosselstrecke bzw. das Drosselelement geändert bzw. angepasst werden. Das eigentliche Ventil mit seinen beiden vorgegebenen Schaltstellungen kann hierzu unverändert beibehalten werden.

Nach der Erfindung ist das Absperrelement des Ventils von einem Ventilantrieb betätigbar, welcher ein Betätigungselement umfasst, welches zwei temperaturabhängige stabile Schaltstellungen aufweist, wobei das Betätigungselement bei Überschreiten einer Schwellentemperatur das Absperrelement aus der Gesperrt-Stellung in die Geöffnet-Stellung und bei Unterschreiten der Schwellentemperatur aus der Geöffnet-Stellung in die Gesperrt-Stellung bewegt.

Das Betätigungselement kann als gerades oder gekrümmtes plattenförmiges Element ausgebildet sein, welches so angeordnet ist, dass es mit seiner der Öffnung des Ventilsitzes zugewandten Oberfläche den Abgabepfad für die Substanz begrenzt. Ein derartiges Betätigungselement kann beispielsweise aus einem Bimetall oder einer Formgedächtnis-Legierung (Memory-Metall; Shape Memory Alloy (SMA)) bestehen. Das Bimetall kann als gekrümmter Teller ausgebildet sein, so dass sich ein "Knackfrosch"-Effekt ergibt, wenn das Betätigungselement eine vorgegebene Schwellentemperatur überschreitet. All diese Ausführungsformen ermöglichen das Übertragen relativ hoher Kräfte mit geringer Initialisierungsenergie. Bei einer Ausbildung des Betätigungselements als Memory-Metallscheibe wird diese in geeigneter Weise vorgeprägt.

Das Gehäuse ist erfindungsgemäß zweiteilig ausgebildet, wobei ein erstes Gehäuseteil als Trägereinheit für die abzugebende Substanz fungiert und ein zweites Gehäuseteil die Ventileinheit und die Steuereinheit (Steuer- und Antriebseinheit) umfasst. Durch diese Modularität können unterschiedliche Trägereinheiten für unterschiedliche Substanzen mit verschiedenen Steuer- und Antriebseinheiten bedarfsweise miteinander verbunden werden.

Nach einer Ausgestaltung der Erfindung kann das Absperrelement in der Gesperrt-Stellung von der mit Druck beaufschlagten abzugebenden Substanz dichtend gegen einen Ventilsitz der Ventileinheit gedrückt werden. Hierdurch erübrigen sich aufwändige Ventilsteuerungen oder Maßnahmen durch Abdichtung des Ventils in der Gesperrt-Stellung. Selbstverständlich kann zusätzlich ein federndes Rückstellelement vorgesehen sein, welches das Absperrelement zusätzlich in Richtung auf den Ventilsitz beaufschlagt, um das Absperrelement nach seinem Bewegen in die Geöffnet-Stellung und dem Deaktivieren des Ventilantriebs schneller und/oder mit höherer Kraft wieder in die Gesperrt-Stellung zurück zu bewegen.

Das Betätigungselement kann die Öffnung des Ventilsitzes durchgreifen und in der Geöffnet-Stellung das Absperrelement gegen den Druck der Substanz vom Ventilsitz abheben. Dies ermöglicht das direkte Abheben des Absperrelements vom Ventilsitz mit geringer Kraft, ohne dass eine Kraftumlenkung und damit zusätzlicher mechanischer Aufwand erforderlich wäre.

Nach einer bevorzugten Ausführungsform der Erfindung ist an der dem Ventilsitz abgewandten Seite des Betätigungselements ein von der Steuereinheit aktivierbares Heizelement für das Erwärmen des Betätigungselements vorgesehen. Zur Abgabe der Substanz wird das thermisch aktivierbare Betätigungselement durch das Heizelement erhitzt und damit in seine Form oberhalb der Grenztemperatur gebracht. Die Grenztemperatur zur Formänderung liegt dabei vorzugsweise deutlich über der Körpertemperatur, bei etwa 45-60°C. Die thermische Hysterese muss in jedem Fall klein genug sein, um ein Zurückschnappen bzw. Zurückbewegen des Betätigungselements in seine Stellung unterhalb der Grenztemperatur (ohne Heizung) sicherzustellen.

Nach einer Ausgestaltung der Erfindung kann an der dem Ventilsitz zugewandten Seite des Betätigungselements eine Isolierschicht angeordnet sein, die sich vorzugsweise über den gesamten Bereich des Betätigungselements erstreckt, der den Abgabepfad begrenzt. Hierdurch wird eine Erwärmung der abzugebenden Substanz vermieden, die durch den Abgabepfad strömt und mit dem zur Aktivierung erhitzten Betätigungselement in Berührung käme, wenn dieses nicht mit einer Isolierschicht versehen wäre. Die Isolierschicht besteht dabei vorzugsweise aus einem Material, das nicht mit der Substanz reagiert oder diese beeinträchtigt. Gleichzeitig kann die Isolierschicht das Betätigungselement vor einer Beeinträchtigung durch die Substanz, insbesondere vor Korrosion schützen und damit Fehlfunktionen der elektronischen Pille vermeiden.

Die Ventileinheit kann ein Kugelventil umfassen, wodurch sich eine einfache und kostengünstige Herstellung ergibt.

Vorzugsweise sind im Gehäuse Mittel zur Erzeugung eines konstanten Drucks zur Beaufschlagung der abzugebenden Substanz vorgesehen, die den konstanten Druck unabhängig vom Volumen, die die Substanz im Behälter beansprucht, aufrecht erhalten. Hierdurch ergibt sich ein im Wesentlichen konstanter Massenstrom nach dem Öffnen des Ventils.

Die Mittel zur Erzeugung eines konstanten Drucks umfassen vorzugsweise ein Flüssiggas, welches bei Körpertemperatur einen definierten Druck erzeugt und unabhängig vom Volumen der Substanz bei der Abgabe der Substanz aufrecht erhält, wobei der Gasdruck die Substanz mittelbar beaufschlagt. Das Flüssiggas bzw. die Flüssigkeit wird dabei so gewählt, dass der Dampfdruck bei der Einsatztemperatur der Pille, beispielsweise bei der menschlichen Körpertemperatur von 37°C, den gewünschten Vordruck von beispielsweise 0.5 bis 3 bar erzeugt. Je nach Substanz und gewünschtem Vordruck können dabei unterschiedliche, vorzugsweise inerte Druck-Flüssigkeiten, wie beispielsweise Freone, verwendet werden.

Nach der bevorzugten Ausführungsform der Erfindung ist im Gehäuse eine deformierbare, vorzugsweise flexible Hülle für die Substanz vorgesehen, so dass im Gehäuse außerhalb der Hülle das Flüssiggas so vorgesehen sein kann, dass der Dampfdruck die Hülle beaufschlagt.

Vorzugsweise sind die Mittel zur Erzeugung eines konstanten Drucks bzw. die flexible Hülle und das Flüssiggas ebenfalls in der Trägereinheit vorgesehen.

Nach einer Ausgestaltung der Erfindung weist die Trägereinheit als Verschluss für die Substanz ein Septum auf, welches im zusammengesetzten Zustand der beiden Gehäuseteile von einer Nadel oder Kanüle durchstochen ist, die am zweiten Gehäuseteil vorgesehen ist und deren Kanal mit dem Absperrelement der Ventileinheit verschließbar bzw. öffenbar ist. Hierdurch kann auf einfache Weise das Ventil mit dem zunächst geschlossenen Volumen für die abzugebende Substanz verbunden werden. Die Verbindung kann durch eine geeignete Materialwahl für das Septum und durch eine geeignete Dimensionierung für die Nadel bzw. die Kanüle (insbesondere deren Durchmesser) im interessierenden Bereich sogar druckdicht gestaltet werden.

Die Trägereinheit kann in der Produktion durch den Port bzw. das Septum ebenfalls mit Hilfe einer dünnen Nadel auf einfache Weise befüllt werden. Dabei kann auch die Druckflüssigkeit zuvor in die Trägereinheit eingebracht und die Trägereinheit versiegelt werden. Dies erfolgt vorzugsweise bei einer Temperatur, bei der das Gas noch flüssig ist und nicht nennenswert verdampft.

Die Abgabeöffnung im Gehäuse kann nach einer weiteren Ausführungsform der Erfindung in der Gesperrt-Stellung der Ventileinheit verschlossen sein und in der Geöffnet-Stellung der Ventileinheit durch den im Abgabepfad entstehenden Überdruck geöffnet werden. Dies lässt sich beispielsweise dadurch erreichen, dass die Gehäuseteile miteinander verrastet werden, wobei ein Gehäuseteil das andere im drucklosen Zustand des Abgabepfads dichtend umgreift. Der Abgabepfad kann dabei bis an diesen Gehäusebereich geführt und so flexibel ausgebildet sein, dass die abzugebende Substanz durch die Verbindungsstelle zwischen den beiden Gehäuseteilen hindurchgedrückt wird.

Die Steuereinheit umfasst vorzugsweise eine Sende- und Empfangseinheit, die unidirektional oder bidirektional mit einer körperexternen Steuereinheit kommunizieren kann. Hierdurch kann insbesondere der Abgabevorgang ferngesteuert ausgelöst und erforderlichenfalls auch der zeitliche Verlauf ferngesteuert werden.

Weitere Ausführungsformen der Erfindung ergeben sich aus den Unteransprüchen.

Die Erfindung wird nachstehend anhand eines in der Zeichnung dargestellten Ausführungsbeispiels näher erläutert. In der Zeichnung zeigen:
- Fig. 1: einen schematischen Längsschnitt durch eine Ausführungsform einer elektronischen Pille nach der Erfindung;
- Fig. 2: einen Längsschnitt durch die Substanzträgereinheit der elektronischen Pille in Fig. 1;
- Fig. 3: einen Längsschnitt durch die Steuer- und Antriebseinheit der elektronischen Pille in Fig. 1;
- Fig. 4: eine vergrößerte, schematische Seitenansicht der Antriebseinheit der Steuer- und Antriebseinheit in Fig. 3; und
- Fig. 5: eine Draufsicht der Antriebseinheit der Steuer- und Antriebseinheit in Fig. 4.

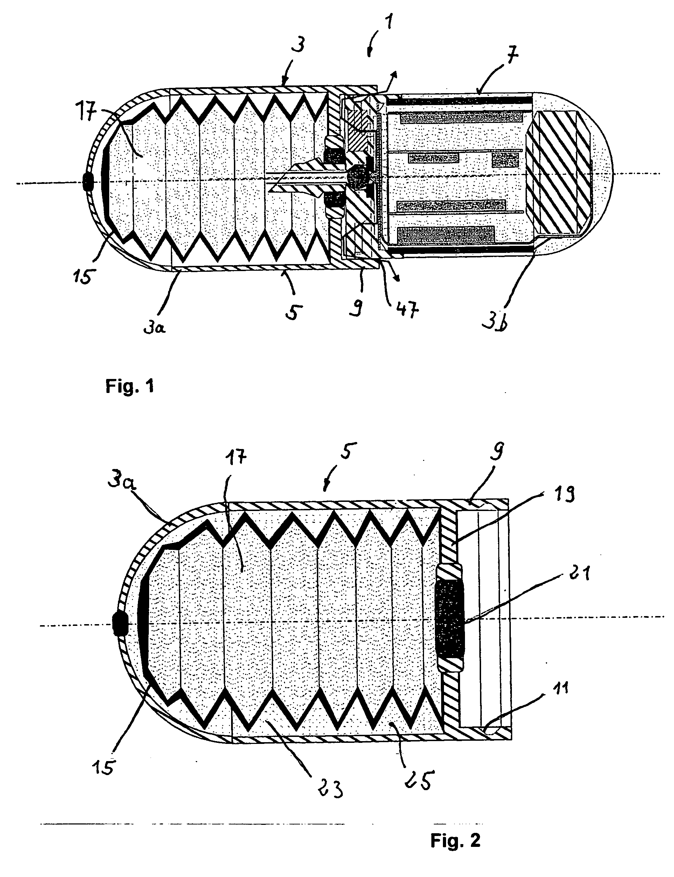

Ein Ausführungsbeispiel einer elektronischen Pille nach der Erfindung ist in Fig. 1 dargestellt. Diese elektronische Pille 1 umfasst ein Gehäuse 3, welches aus einem ersten Gehäuseteil 3a und einem zweiten Gehäuseteil 3b besteht. Das Gehäuse 3 ist in seiner Größe und seiner Form so gewählt, dass es in einen menschlichen oder tierischen Körper eingebracht, insbesondere geschluckt werden kann.

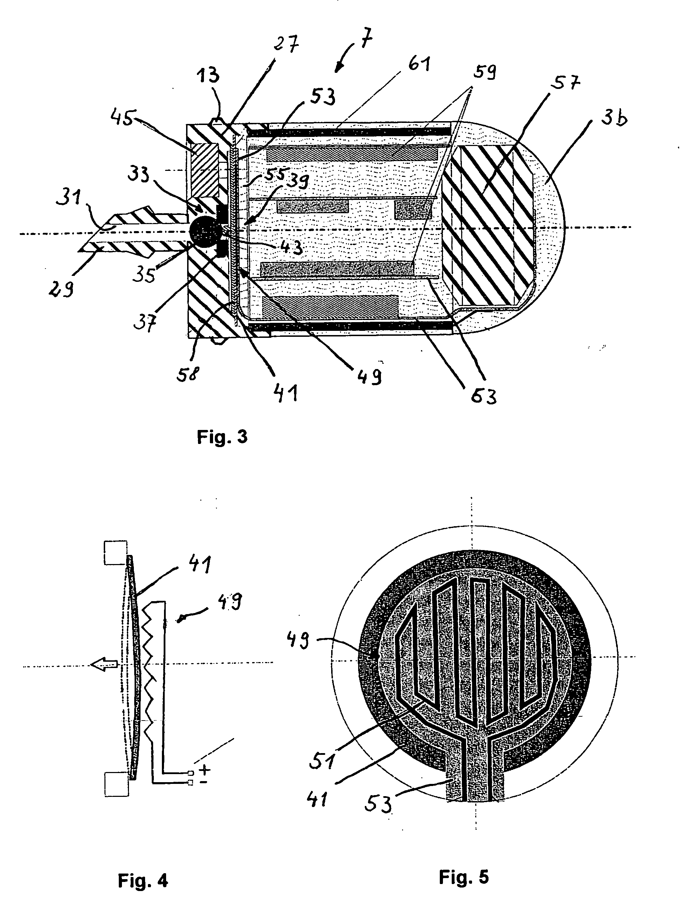

Das erste Gehäuseteil ist einer Trägereinheit 5 für eine zu verabreichende, d.h. in einen menschlichen oder tierischen Körper einzubringende Substanz zugeordnet. Die Trägereinheit 5 ist mit einer Steuer und Antriebseinheit 7 verbunden, welche das zweite Gehäuseteil 3b umfasst. Das erste Gehäuseteil 3a weist hierzu an seiner dem zweiten Gehäuseteil 3b zugewandten Seite einen umlaufenden Bund 9 auf, welcher das zweite Gehäuseteil 3b umgreift. Wie aus den Fig. 2 und 3 ersichtlich, kann eine Verrastung der beiden Gehäuseteile 3a, 3b mittels einer in der Innenwandung des umlaufenden Bunds 9 des ersten Gehäuseteils 3a vorgesehenen Nut 11 und einer damit zusammenwirkenden an der Außenwandung des vorderen Bereichs des zweiten Gehäuseteilis 3b vorgesehenen umlaufenden Rasterhebung 13 vorgesehen sein. Selbstverständlich können die beiden Gehäuseteile 3a, 3b jedoch auch auf jede geeignete andere Weise miteinander verbunden werden, beispielsweise über einen einfachen Presssitz, mittels eines Bajonettverschlusses oder durch Verschweißen der vorzugsweise aus einem geeigneten Kunststoff bestehenden Gehäuseteile. Vorzugsweise ist die Verbindung so ausgestaltet, dass eine feste, unlösbare Verbindung erfolgt, um im Körper eine unbeabsichtigte Trennung der Gehäuseteile zu vermeiden.

Im ersten Gehäuseteil 3a der Trägereinheit 5 ist eine flexible Hülle 15 für die zu verabreichende Substanz 17, beispielsweise ein Medikament, vorgesehen. Die Substanz ist vorzugsweise flüssig oder zumindest ausreichend viskos. Zur Verabreichung nicht flüssiger Wirkstoffe können diese in eine geeignet gewählte Trägerflüssigkeit eingebracht werden. Wie aus Fig. 2 ersichtlich, kann die flexible Hülle 15 faltenbalgartig und einseitig offen ausgebildet und mit ihrem vorderen Bereich mit einer vorderen Wandung 19 des ersten Gehäuseteils 3a verbunden sein, welche koaxial und druckdicht ein Septum 21 umschließt. Auf diese Weise wird ein dichter Raum zur Aufnahme der Substanz 17 geschaffen, der durch die flexible Hülle 15 und gegebenenfalls einen Teil der Wandung 19 sowie dem Septum begrenzt ist. Die Hülle 15 kann jedoch auch geschlossen ausgebildet und in entsprechenden Bereichen flächig oder zumindest in Teilflächen mit der Wandung 19 und/oder dem Septum 21 verbunden sein.

Im Zwischenraum 23 zwischen der flexiblen Hülle 15 und der Innenwandung des ersten Gehäuseteils 3a ist ein Flüssiggas 25 eingebracht. Hierunter wird im Rahmen der vorliegenden Beschreibung eine Kombination aus einer Flüssigkeit und der durch diese erzeugten gasförmigen Phase verstanden, die bei der Einsatztemperatur der elektronischen Pille 1, also der Normaltemperatur des betreffenden Körpers (beispielsweise der Normaltemperatur von 37°C des menschlichen Körpers), einen ausreichenden Druck (Dampfdruck der Flüssigkeit) erzeugt, um bei einer Öffnung eines Abgabepfads die Substanz 17 aus der elektronischen Pille austreiben zu können. Die Flüssigkeit wird so gewählt, dass ein konstanter Überdruck von etwa 0,3 bis 3 bar erzeugt wird, wobei die höheren Drücke für hoch viskose Substanzen und die niedrigeren Drücke für sehr niedrigviskose, dünnflüssige Substanzen verwendet werden.

Das Einbringen der Substanz 17 in die flexible Hülle 15 kann beispielsweise dadurch erfolgen, dass das Septum und gegebenenfalls die flexible Hülle 15 mit einer Füllnadel (nicht dargestellt) durchstochen und eine definierte Menge der Substanz 17 über die Füllnadel eingebracht wird. Der Füllvorgang kann nach dem fertigen Herstellen der Trägereinheit 5, d.h. auch nach dem Einbringen des Flüssiggases 25, erfolgen. Die Temperatur der Umgebung bzw. der Trägereinheit 5 und damit des Flüssiggases 25 kann hierzu so niedrig gewählt werden, dass der Dampfdruck auf die flexible Hülle 15 so gering ist, das der Füllvorgang mit relativ geringem Druck durchgeführt werden kann. Der Raum 23 für das Flüssiggas 25 ist nach Befüllung hermetisch abgeschlossen. Hierdurch steht die Substanz 17 unter dem konstanten (temperaturabhängigen) Vordruck, unabhängig davon, wie weit der Behälter bereits entleert wurde.

Die Steuer- und Antriebseinheit 7 umfasst einen beispielsweise aus Kunststoff gespritzten Basiskörper 27, der einen Teil des zweiten Gehäuseteils 3b bildet und in welchem koaxial eine Nadel 29 angeordnet ist, welche über die Vorderseite des zweiten Gehäuseteils 3b so weit hinausragt, dass die Nadel 29 beim Zusammensetzen der Steuer- und Antriebseinheit 7 mit der Trägereinheit 5 durch das Septum 21 sticht und so der Kanal 31 der Nadel 29 mit dem Innenraum verbunden wird, in dem die Substanz 17 aufgenommen ist. Die Nadel 29 kann, wie in Fig. 3 dargestellt, mit einem vorzugsweise umlaufenden Widerhaken 33 versehen sein, der zusätzlich zum Haltemechanismus zwischen den Gehäuseteilen 3a, 3b ein unbeabsichtigtes Lösen der Baugruppen 5, 7 verhindert. Zudem kann die nach dem Verbinden der Baugruppen 5, 6 an der Innenwandung des Septums 21 anliegende Schulter des Widerhakens 31 die Dichtwirkung verbessern.

Im Basiskörper 27 ist eine Ventileinheit vorgesehen, die eine ein Absperrelement bildende Kugel 35, die beispielsweise aus Glas oder Metall bestehen kann, einen Ventilsitz 37, der ebenfalls aus Glas oder Metall bestehen kann, und eine Ventilantriebseinheit 39 umfasst. Die Ventilantriebseinheit 39 umfasst ein leicht tellerartig gekrümmtes, plattenförmiges Betätigungselement 41, das zumindest teilweise aus einem Bimetall oder einem Memory-Metall bestehen kann. Das Betätigungselement 41 erhält durch die tellerartige Krümmung zwei temperaturabhängige stabile Zustände: Unterhalb einer Schwellentemperatur wird der eine stabile Zustand und oberhalb der Schwellentemperatur der andere stabile Zustand eingenommen. Die Zustände können durch die Wahl der Geometrie und der Materialien und gegebenenfalls mittels eines Einprägevorgangs so definiert werden, dass sich beispielsweise die in Fig. 4 dargestellten Zustände des Betätigungselements 41 ergeben. Unterhalb der Schwellentemperatur ist das Betätigungselement leicht konvex in Richtung auf das äußere Ende der Steuer- und Antriebseinheit gebogen (erster stabiler Zustand) und oberhalb der Schwellentemperatur leicht konvex in Richtung zur Nadel 29 (zweiter stabiler Zustand). Der Übergang zwischen den stabilen Zuständen erfolgt durch die tellerartige Krümmung sprungartig ("Knackfroscheffekt"). Im Zentrum des Betätigungselements 41 kann der maximale Verstellweg, von beispielsweise ca. 0,3 mm abgegriffen werden, der die Kugel 35 des Ventils öffnet.

An seiner der Kugel 35 zugewandten Seite ist am Betätigungselement 41 ein Stößel bzw. eine Nase 43 vorgesehen, die den ringförmigen Ventilsitz 37 durchgreift. Das Betätigungselement 41 ist im Wesentlichen senkrecht zur Längsachse der Nadel 29 im Basiskörper 27 derart gehalten, dass die Nase 43 im ersten stabilen Zustand (in Fig. 4 ausgezogen dargestellt) die Kugel nicht oder allenfalls mit geringer Kraft beaufschlagt, so dass das Ventil geschlossen ist. Im zweiten stabilen Zustand (in Fig. 4 gestrichelt dargestellt) beaufschlagt die Nase 43 die Kugel und drückt sie um eine relativ kleine Wegstrecke gegen den Druck der Substanz 17 aus dem Ventilsitz, so dass das Ventil geöffnet wird.

Das Schließen des Ventils erfolgt bei deaktiviertem Betätigungselement (d.h. das Betätigungselement befindet sich im ersten stabilen Zustand) durch den Druck, den die Substanz auf die Kugel 5 ausübt. Zusätzlich kann selbstverständlich auch ein federndes Element vorgesehen sein, welches die Kugel 35 zusätzlich in Richtung auf den Ventilsitz 37 beaufschlagt. Diese Ventilkonstruktion schließt präzise und gewährleistet eine hervorragende Dichtigkeit.

Als Schwellentemperatur wird ein Wert gewählt, der ausreichend höher ist, als die Normaltemperatur des Körpers, in dem die elektronische Pille verwendet werden soll. Beispielsweise kann die Schwellentemperatur in einem Bereich von 5 bis 15 K über der Normaltemperatur des Körpers gewählt werden. Für die Anwendung im menschlichen Körper kann beispielsweise eine Schwellentemperatur von 45°C gewählt werden.

Im zweiten Gehäuseteil 3b bzw. dem Basiskörper 27 ist ein fluidischer Abgabepfad für die Substanz 17 ausgebildet , der sich über den Kanal 31 der Nadel 29 und über die Bohrung im ringförmigen Ventilsitz 37 bis zu einem Drosselelement 45 erstreckt. Bei verbundenen Gehäuseteilen 3a, 3b wird der Abgabepfad durch den Zwischenraum zwischen der Stirnseite des Basiskörpers 27 bzw. des zweiten Gehäuseteils 3b und der Wandung 19 des ersten Gehäuseteils 3a fortgeführt und erstreckt sich bis an den umlaufenden Bund 9. Dieser kann axiale Nuten (nicht dargestellt) aufweisen, die den Abgabepfad bis zu Abgabeöffnungen 47 verlängern, über welche die Substanz 17 austreten kann. Die axialen Nuten können selbstverständlich auch oder zusätzlich im Umfang des Basiskörpers 27 vorgesehen sein, der vom Bund 9 umgriffen wird.

Anstelle der Nuten kann der Bund 9 des ersten Gehäuseteils 3a zumindest in Teilbereichen so flexibel ausgebildet sein, dass er durch den Druck der Substanz 17 bei geöffnetem Ventil 35, 37 geringfügig vom Umfang des Basiskörpers 27 bzw. dem zweiten Gehäuseteil 3b abgehoben wird, so dass die Substanz über den so erzeugten Spalt austreten kann.

Die Drosselstrecke ist bei der in den Figuren dargestellten Ausführungsform als Drosselelement 45 ausgebildet, welches durch ein poröses Filterelement oder Kapillarfilterelement realisiert ist. Die Drosselwirkung ergibt sich damit praktisch ausschließlich durch den fluidischen Widerstand des porösen Filterelments , wobei die verhältnismäßig deutlich geringeren fluidischen Widerstände der übrigen Teile des Abgabepfads gegenüber dem hohen fluidischen Widerstand des Drosselelements vernachlässigbar sind. Selbstverständlich kann jedoch die Drosselstrecke anstelle eines Filterelements, welches gleichzeitig auch das Eindringen von Stoffen aus der Umgebung, insbesondere von Körperflüssigkeiten in das Pilleninnere verhindert, auch als einfache Verengung des Abgabepfades realisiert sein.

Für das Steuern des Betätigungselements 41 ist eine Heizeinheit 49 vorgesehen, die in unmittelbarer Nachbarschaft auf der Rückseite des Betätigungselements 41 (d.h. auf der dem Ventil 35, 37 abgewandten Seite des Betätigungselements 41) in der Steuer- und Antriebseinheit 7 vorgesehen ist. Die Heizeinheit kann, wie in Fig. 3 und 5 ersichtlich, durch einen oder mehrere Heizleiter 51, die auf einer flexiblen Verdrahtungsplatine (Polyamid-Verdrahtung) 53 integriert sind und die das Betätigungselement 41 unmittelbar berühren, realisiert sein. Auf der Rückseite der flexiblen Verdrahtungsplatine 53 ist ein elastisches Andruckelement 55, beispielsweise ein elastischer Schaumverguss, vorgesehen, welches die flexible Verdrahtungsplatine mit dem Heizleiter 51 in jeder Position flächig an das Betätigungselement 41 andrückt. Gleichzeitig gewährleistet das Andruckelement 55 eine ausreichende Wärmedämmung zum restlichen Teil der Steuer- und Antriebseinheit 7, insbesondere den übrigen elektronischen Komponenten. Die Heizeinheit bzw. der oder die Heizleiter sind so ausgeführt, dass ein unmittelbarer Betrieb mit einer Batterie 57 erfolgen kann, wobei das Schalten der Heizung, d.h. das Ansteuern der Heizeinheit 49 durch einen Prozessor erfolgen kann.

Auch an der Vorderseite des Betätigungselements 41 kann eine Isolierschicht 58 vorgesehen sein, um eine unzulässige Temperatureinwirkung auf die abzugebende Substanz 17 zu vermeiden, wenn das Betätigungselement 41 über die Schwellentemperatur erhitzt wird und das Ventil 35, 37 öffnet.

Eine so aufgebaute Ventileinheit 33 schaltet im Sekundenbereich bei sehr geringer Steuerleistung. Wesentlicher Vorteil gegenüber Piezoantrieben und elektromagnetischen Antrieben ist die extrem kleine Bauweise des Antriebs bei gleichzeitig sehr niedrigen Fertigungskosten.

Nach dem Aktivieren der Heizeinheit 49 und dem Öffnen des Ventils 35, 37 kann die abzugebende Substanz 17 über das Drosselelement 45 in den Teil des Abgabepfades eintreten, der durch den Zwischenraum zwischen der Trägereinheit 5 und der Steuer- und Antriebseinheit gebildet wird. Von da aus wird die Substanz 17 über die Abgabeöffnung, die beispielsweise durch den ausreichend flexiblen oder durchlässigen umlaufenden Bund 9 realisiert sein kann, in den Außenbereich abgegeben.

Als Drosselelement 45 können Kapillarfilter, beispielsweise verdichtete Keramikkörper mit Poren dienen, z.B. Glas-Keramik-Fritten, die den Durchfluss der Substanz 17, beispielsweise des Medikaments, auf definierbare Flussraten begrenzen. Die Menge der abgegebenen Substanz 17 ist damit durch die Zeit, die das Ventil 35, 37 geöffnet ist, kontrollierbar. Das Drosselelement 45 kann beispielsweise so dimensioniert werden, dass das gesamte abzugebende Substanzvolumen 1000 sec benötigt, um bei dem gegebenen konstanten Vordruck die Trägereinheit 5 zu leeren, z.B. bei 1 ml Volumen ein Strom von 1 µl/sec. Auch bei einem relativ langsam schaltenden Ventil 35, 37 ist damit die Dosierung sehr genau kontrollierbar.

Die Trennung von Drosselstrecke und Ventil 35, 37 ermöglicht bei den winzigen Dimensionen eine genaue Dimensionierung der elektronischen Pille 1 bezüglich des Durchflusses. Durch den hohen (konstanten) Vordruck und das nichtlineare Verhalten der Drosselstrecke ist das System auf Gegendruck sehr unempfindlich, d.h. Druckverhältnisse am Einsatzort der elektronischen Pille 1 beeinflussen die Flussrate der Substanz 17 während des Abgebens praktisch nicht. Dies ist ein Vorteil gegenüber Lösungen mit Pumpen an dieser Stelle, die alle eine mehr oder weniger hohe Empfindlichkeit auf Gegendruck aufweisen und deshalb in der Dosierung ungenauer sind. Zudem wird nur geringe Energie zum Öffnen und Schließen des Ventils benötigt, da die Transportenergie aus dem Vordruck des Behälters stammt und nicht elektrisch aufgebracht werden muss.

Im Gehäuseteil 3b sind des Weiteren die elektronischen Komponenten 59 der Steuer- und Antriebseinheit auf einer flexiblen Platine 63 angeordnet, die aufgewickelt in den durch eine Antennenspule 61 gebildeten Hohlraum gesteckt ist. Die Platine 63 kann, wie in Fig. 3 dargestellt, einstückig mit der flexiblen Platine 53 verbunden sein, auf welcher der Heizleiter 51 angeordnet ist. Die Batterie 57 kann direkt mit der Platine 63 verbunden sein. Die Wickeltechnik ermöglicht, die elektronischen Bauteile klassisch planar zu montieren, was ebenfalls die Herstellkosten senkt. Bei sehr hoher Integration der Elektronik kann auch ein scheibchenförmiger Aufbau erfolgen. Der gesamte Elektronikteil wird nach Fertigung und Prüfung mit einem medizinisch zugelassenen Harz vergossen, welches auch resistent gegenüber den Körpersäften ist. Hierdurch wird das Gehäuseteil 3b gebildet.

Die Steuer- und Antriebseinheit 7 mit der vorstehend erläuterten Bimetall-Aktuator-Ventileinheit 33 ist sehr einfach und kostengünstig produzierbar und kann auf alle wesentlichen Substanzen optimiert werden, insbesondere durch das Einsetzen eines geeigneten Drosselelements 45, ohne die sonstige Ventileinheit verändern zu müssen. Die Bimetall-Ausführung verfügt über eine sehr geringe Hysterese mit schnellen Schließ- und Öffnungszeiten, wobei die erforderliche Energie zur Ventilöffnung gering ist. Die Ausführung der Ventileinheit 33 verfügt über das Potential einer Mikrominiaturisierung, was für regelmäßig einzunehmende Medikamente marktentscheidend ist und nur so eine Einmalverwendung rechtfertigen kann.

Damit schafft die Erfindung ein Therapiesystem, das ganz neue Anwendungen in der Krankenversorgung ermöglicht. Die Genauigkeit und Sicherheit der Dosierung in Volumen und Zeitpunkt ermöglicht auch die orale Therapierung chronischer Krankheiten wie z.B. Diabetes Mellitus, was mit den bisherigen Systemen nicht möglich war.

Die Medikamentenbehälter bzw. Trägereinheiten können in großen Serien bei pharmazeutischen Unternehmen mit unterschiedlichen Substanzen gefertigt und gelagert werden. Die Kontrolleinheiten können ebenfalls in großen Serien von Geräteherstellern gefertigt werden und können hinsichtlich ihres Aufbaus im Wesentlichen identisch sein und sich nur durch die dem Verwendungszweck angepasste Programmierung und/oder durch ein anderes Drosselelement unterscheiden.

## Patentansprüche

1. Elektronische Pille zur steuerbaren Abgabe einer Substanz, insbesondere eines Medikaments, in einem menschlichen oder tierischen Körper,
(a) mit einem Gehäuse (3), in welchem die abzugebende Substanz (17) aufgenommen und in welchem eine Abgabeöffnung (47) zur Abgabe der Substanz (17) vorgesehen ist, wobei die Substanz (17) zur Abgabe aus dem Gehäuse (3) mit einem vorbestimmten Druck beaufschlagbar ist,
(b) mit einer elektronischen Steuereinheit (53, 59, 61, 63) und
(c) mit einer im Verlauf eines Abgabepfades angeordneten Ventileinheit (33), welche von der Steuereinheit (53, 59, 61, 63) von einer Geöffnet-Stellung in eine Geschlossen-Stellung steuerbar ist,
(d) **dadurch gekennzeichnet,**
(e) **dass** das Gehäuse (3) zweiteilig ausgebildet ist und ein erstes Gehäuseteil (3a) als Trägereinheit (5) für die abzugebende Substanz (17) ausgebildet ist und ein zweites Gehäuseteil (3b) die Ventileinheit (33) und die Steuereinheit (53, 59, 61, 63) umfasst,
(f) **dass** im Gehäuse (3) im Verlauf des Abgabepfades für die abzugebende Substanz (17) eine Drosselstrecke (45) vorgesehen ist, welche als Drosselelement (45) ausgebildet ist oder welche ein Drosselelement (45) umfasst,
(g) **dass** das Drosselelement (45) eine definierte Porosität aufweist,
(h) **dass** ein Absperrelement (35) der Ventileinheit (33) von einer Ventilantriebseinheit (39) betätigbar ist, welche ein Betätigungselement (41) umfasst, welches zwei temperaturabhängige stabile Schaltstellungen aufweist, wobei das Betätigungselement (41) bei Überschreiten einer Schwellentemperatur das Absperrelement (35) aus der Gesperrt-Stellung in die Geöffnet-Stellung und bei Unterschreiten der Schwellentemperatur aus der Geöffnet-Stellung in die Gesperrt-Stellung bewegt, und
(i) **dass** das Betätigungselement (41) als gerades oder gekrümmtes plattenförmiges Element ausgebildet ist, welches so angeordnet ist, dass es mit seiner der Öffnung eines Ventilsitzes (37) zugewandten Oberfläche den Abgabepfad für die Substanz (17) begrenzt.

2. Elektronische Pille nach Anspruch 1, **dadurch gekennzeichnet, dass** das Absperrelement (35) der Ventileinheit (33) in der Gesperrt-Stellung von der mit Druck beaufschlagten abzugebenden Substanz dichtend gegen einen Ventilsitz (37) der Ventileinheit (33) gedrückt ist.

3. Elektronische Pille nach Anspruch 2, **dadurch gekennzeichnet, dass** das Betätigungselement (41) die Öffnung des Ventilsitzes (37) durchgreift und in der Geöffnet-Stellung das Absperrelement (35) gegen den Druck der Substanz (17) vom Ventilsitz (37) abhebt.

4. Elektronische Pille nach Anspruch 2 oder 3, **dadurch gekennzeichnet, dass** an der dem Ventilsitz (37) abgewandten Seite des Betätigungselement (41) eine Heizeinheit (49) für das Erwärmen des Betätigungselements (41) vorgesehen ist, welches von der Steuereinheit (53, 59, 61, 63) aktivierbar ist.

5. Elektronische Pille nach Anspruch 4, **dadurch gekennzeichnet, dass** an der dem Ventilsitz (37) zugewandten Seite des Betätigungselements (41) eine Isolierschicht (58) angeordnet ist, die sich vorzugsweise über den gesamten Bereich des Betätigungselements (41) erstreckt, der den Abgabepfad begrenzt.

6. Elektronische Pille nach einem der vorhergehenden Ansprüche, **dadurch gekennzeichnet, dass** das Ventil (35, 37) als Kugelventil ausgebildet ist.

7. Elektronische Pille nach einem der vorhergehenden Ansprüche, **dadurch gekennzeichnet, dass** die Drosselstrecke (45) zwischen dem Absperrelement (35) der Ventileinheit (33) und der Abgabeöffnung (47) vorgesehen ist.

8. Elektronische Pille nach einem der vorhergehenden Ansprüche, **dadurch gekennzeichnet, dass** im Gehäuse (3) Mittel (15, 25) zur Erzeugung eines konstanten Drucks zur Beaufschlagung der abzugebenden Substanz (17) vorgesehen sind, die den konstanten Druck unabhängig vom Volumen, die die Substanz (17) im Behälter (3) beansprucht, aufrecht erhalten.

9. Elektronische Pille nach Anspruch 8, **dadurch gekennzeichnet, dass** die Mittel (15, 25) zur Erzeugung eines konstanten Drucks ein Flüssiggas (25) umfassen, welches bei der Einsatztemperatur einen definierten Druck erzeugt und unabhängig vom Volumen der Substanz (17) bei der Abgabe der Substanz (17) aufrecht erhält, wobei der Gasdruck die Substanz (17) mittelbar beaufschlagt.

10. Elektronische Pille nach Anspruch 9, **dadurch gekennzeichnet, dass** im Gehäuse (3) eine deformierbare, vorzugsweise flexible Hülle (15) für die Substanz (17) vorgesehen ist und dass im Gehäuse (3) außerhalb der flexiblen Hülle (15) das Flüssiggas (25) vorgesehen ist.

11. Elektronische Pille nach einem der Ansprüche 8 bis 10, **dadurch gekennzeichnet, dass** die Mittel (15, 25) zur Erzeugung eines konstanten Drucks bzw. die flexible Hülle (15) und das Flüssiggas (25) in der Trägereinheit (5) vorgesehen sind.

12. Elektronische Pille nach einem der vorhergehenden Ansprüche, **dadurch gekennzeichnet, dass** die Trägereinheit (5) als Verschluss für die Substanz (17) ein Septum (21) aufweist, welches im zusammengesetzten Zustand der beiden Gehäuseteile (3a, 3b) von einer Nadel (29) oder Kanüle durchstochen ist, die am zweiten Gehäuseteil (3b) vorgesehen ist und deren Kanal (31) mit dem Absperrelement (35) der Ventileinheit (33) verschließbar bzw. öffenbar ist.

## Claims

1. An electronic pill for dispensing a substance, in particular a drug, in a human or animal body in a controllable manner,
(a) having a housing (3), in which the substance (17) that is to be dispensed is accommodated and in which a dispensing opening (47) for dispensing the substance (17) is provided, wherein a predetermined pressure can be applied to the substance (17) for the dispensing from the housing (3),
(b) having an electronic control unit (53, 59, 61, 63), and
(c) having a valve unit (33) which is arranged in the course of a dispensing path and can be controlled by the control unit (53, 59, 61, 63) from an opened position into a closed position,
(d) **characterised**
(e) **in that** the housing (3) is formed in two portions, and a first housing portion (3a) is formed as a carrier unit (5) for the substance (17) that is to be dispensed, and a second housing portion (3b) comprises the valve unit (33) and the control unit (53, 59, 61, 63),
(f) **in that** provided in the housing (3) in the course of the dispensing path for the substance (17) that is to be dispensed there is a throttle section (45) which is formed as a throttle element (45) or which comprises a throttle element (45),
(g) **in that** the throttle element (45) has a defined porosity,
(h) **in that** a blocking element (35) of the valve unit (33) can be actuated by a valve-drive unit (39) which comprises an actuating element (41) that has two temperature-dependent stable switch positions, wherein the actuating element (41) moves the blocking element (35) out of the blocked position into the opened position when a threshold temperature is exceeded and moves it out of the opened position into the blocked position when there is a fall below the threshold temperature, and
(i) **in that** the actuating element (41) is formed as a straight or curved plate-like element which is arranged in such a way that with its surface that faces the opening of a valve seat (37) it delimits the dispensing path for the substance (17).

2. An electronic pill according to claim 1, **characterised in that** in the blocked position the blocking element (35) of the valve unit (33) is pressed in a sealing manner against a valve seat (37) of the valve unit (33) by the substance that is to be dispensed and to which pressure is applied.

3. An electronic pill according to claim 2, **characterised in that** the actuating element (41) reaches through the opening of the valve seat (37) and in the opened position lifts the blocking element (35) off from the valve seat (37) against the pressure of the substance (17).

4. An electronic pill according to claim 2 or 3, **characterised in that** provided on the side of the actuating element (41) that is remote from the valve seat (37) there is a heating unit (49) to heat the actuating element (41) that can be activated by the control unit (53, 59, 61, 63).

5. An electronic pill according to claim 4, **characterised in that** arranged on the side of the actuating element (41) that faces the valve seat (37) there is an insulating layer (58) which preferably extends over the whole region of the actuating element (41) that delimits the dispensing path.

6. An electronic pill according to one of the preceding claims, **characterised in that** the valve (35, 37) is formed as a ball valve.

7. An electronic pill according to one of the preceding claims, **characterised in that** the throttle section (45) is provided between the blocking element (35) of the valve unit (33) and the dispensing opening (47).

8. An electronic pill according to one of the preceding claims, **characterised in that** in order to generate a constant pressure for application to the substance (17) that is to be dispensed means (15, 25) are provided in the housing (3) that maintain the constant pressure irrespective of the volume taken up by the substance (17) in the container (3).

9. An electronic pill according to claim 8, **characterised in that** the means (15, 25) for generating a constant pressure comprise a liquefied gas (25) that generates a defined pressure at the operating temperature and maintains it irrespective of the volume of the substance (17) during the dispensing of the substance (17), wherein the gas pressure is applied indirectly to the substance (17).

10. An electronic pill according to claim 9, **characterised in that** provided in the housing (3) there is a deformable, preferably flexible, covering (15) for the substance (17), and **in that** the liquefied gas (25) is provided in the housing (3) outside the flexible covering (15).

11. An electronic pill according to one of claims 8 to 10, **characterised in that** the means (15, 25) for generating a constant pressure, or the flexible covering (15) and the liquefied gas (25), are provided in the carrier unit (5).

12. An electronic pill according to one of the preceding claims, **characterised in that** the carrier unit (5) has as a closure for the substance (17) a septum (21) which in the assembled state of the two housing portions (3a, 3b) is pierced by a needle (29) or a cannula that is provided on the second housing portion (3b) and whose channel (31) can be closed or opened by the blocking element (35) of the valve unit (33).

## Revendications

1. Pastille électronique pour la libération contrôlée d'une substance, en particulier d'un médicament, dans un corps humain ou animal,
(a) avec un boîtier (3), dans lequel la substance à libérer (17) est placée et dans lequel une ouverture de libération (47) est prévue pour la libération de la substance (17), la substance (17) pouvant être soumise à une pression prédéterminée pour sa libération hors du boîtier (3),
(b) avec une unité de commande électronique (53, 59, 61, 63) et
(c) avec une unité de soupape (33) agencée sur le tracé d'un trajet de libération, qui peut être commandée par l'unité de commande (53, 59, 61, 63) d'une position ouverte à une position fermée,
(d) **caractérisée en ce que**
(e) le boîtier (3) est constitué de deux parties et une première partie du boîtier (3a) est conçue comme une unité de support (5) pour la substance à libérer (17) et une deuxième partie de boîtier (3b) comprend l'unité de soupape (33) et l'unité de commande (53, 59, 61, 63),
(f) dans le boîtier (3), sur le tracé du trajet de libération, une portion d'étranglement (45) est prévue pour la substance à libérer (17), laquelle portion d'étranglement est conçue comme un élément d'étranglement (45) ou comprend un élément d'étranglement (45),
(g) l'élément d'étranglement (45) présente une porosité définie,
(h) un élément de blocage (35) de l'unité de soupape (33) peut être actionné par une unité d'entraînement de soupape (39), qui comprend un élément d'actionnement (41) qui présente deux positions de commutation stables dépendant de la température, l'élément d'actionnement (41) déplaçant, lors du passage au-dessus d'un seuil de température, l'élément de blocage (35) de la position bloquée à la position ouverte et, lors du passage en dessous du seuil de température, de la position ouverte à la position bloquée, et
(i) l'élément d'actionnement (41) est conçu comme un élément en forme de plaque droit ou incurvé, lequel est agencé de telle sorte qu'il limite, avec sa surface orientée vers l'ouverture d'un siège de soupape (37), le trajet de libération pour la substance (17).

2. Pastille électronique selon la revendication 1, **caractérisée en ce que** dans la position bloquée, l'élément de blocage (35) de l'unité de soupape (33) est comprimé de manière étanche contre un siège de soupape (37) de l'unité de soupape (33) par la substance à libérer soumise à une pression.

3. Pastille électronique selon la revendication 2, **caractérisée en ce que** l'élément d'actionnement (41) saisit l'ouverture du siège de soupape (37) et, dans la position ouverte, soulève l'élément de blocage (35) du siège de soupape (37) contre la pression de la substance (17).

4. Pastille électronique selon la revendication 2 ou 3, **caractérisée en ce que**, sur le côté de l'élément d'actionnement (41) opposé au siège de soupape (37), une unité de chauffage (49) est prévue pour le chauffage de l'élément d'actionnement (41), lequel peut être activé par l'unité de commande (53, 59, 61, 63).

5. Pastille électronique selon la revendication 4, **caractérisée en ce que**, sur le côté de l'élément d'actionnement (41) orientée vers le siège de soupape (37), est agencée une couche d'isolation (58) qui s'étend de préférence sur toute la zone de l'élément d'actionnement (41), laquelle zone limite le trajet de libération.

6. Pastille électronique selon l'une des revendications précédentes, **caractérisée en ce que** la soupape (35, 37) est conçu comme une soupape à bille.

7. Pastille électronique selon l'une des revendications précédentes, **caractérisée en ce que** la portion d'étranglement (45) est prévue entre l'élément de blocage (35) de l'unité de soupape (33) et l'ouverture de libération (47).

8. Pastille électronique selon l'une des revendications précédentes, **caractérisée en ce que**, dans le boîtier (3) sont prévus des moyens (15, 25) générant une pression constante à appliquer sur la substance à libérer (17), qui maintiennent la pression constante indépendamment du volume nécessaire à la substance (17) dans le boîtier (3).

9. Pastille électronique selon la revendication 8, **caractérisée en ce que** les moyens (15, 25) générant une pression constante comprennent un gaz liquide (25), lequel génère une pression défmie pour la température d'entrée en action et maintient cette pression indépendamment du volume de la substance (17) lors de la libération de la substance (17), la pression du gaz comprimant la substance (17) de manière indirecte.

10. Pastille électronique selon la revendication 9, **caractérisée en ce que**, dans le boîtier (3), une gaine (15) déformable, de préférence flexible, est prévue pour la substance (17) et **en ce que**, dans le boîtier (3), la présence du gaz liquide (25) est prévue à l'extérieur de la gaine flexible (15).

11. Pastille électronique selon l'une des revendications 8 à 10, **caractérisée en ce que** les moyens (15, 25) générant une pression constante ou la gaine flexible (15) et le gaz liquide (25) sont prévus dans l'unité de support (5).

12. Pastille électronique selon l'une des revendications précédentes, **caractérisée en ce que** l'unité de support (5) présente, comme fermeture pour la substance (17), un septum (21) qui, dans l'état assemblé des deux parties de boîtier (3a, 3b), est transpercé par une aiguille (29) ou une canule, prévue sur la deuxième partie de boîtier (3b) et dont le canal (31) peut être fermé ou ouvert avec l'élément de blocage (35) de l'unité de soupape (33).
